# EUROPEAN PATENT APPLICATION

(11) **EP 1 657 307 A1**
(43) Date of publication of application: **17.05.2006**
(21) Application number: 04105792.8
(22) Date of filing: 16.11.2004
(51) Int. Cl.: C12N 15/11, A61K 39/00, A61P 35/00, A61P 37/04

(54) **Oligonucleotides that induce the secretion of GM-CSF**

(71) Applicant: IMMUNOTECH S.A., Buenos Aires (AR)
(72) Inventor: Lopez, Ricardo Agustin, BUENOS AIRES (AR)
(74) Representative: Pistolesi, Roberto

(57) **Abstract**

Oligonucleotides having the following general sequence: TTTTTTTTTTTTCATTTTGTTTXPu Wherein X is G, A or T and Pu is A or G, that have the ability to stimulate white cells of the human immune s ystcm in order to produce high amounts of GM -CSF arc disclosed. These oligonucleotides induce the secretion of high amounts of Granulocyte-Macrophage Colony Stimulating Factor (GM -CSF) acting on white human cells. Therefore, they are useful as adjuvant fo r vaccines and for the treatment of tumoral and immunological diseases.

## Description

Oligonucleotides having the following general sequence: TTTTTTTTTTTTCATTTTGTTTXPu Wherein X is G, A or T and Pu is A or G, that have the ability to stimulate white cells of the human immune system in order to produce high amounts of GM -CSF are disclosed.

### BACKGROUND OF THE INVENTION

### The immune system

The major function of the immune system is to protect the host from invading pathogens. A number of different cell types, both antig en-independent and antigen - specific, have evolved to detect and neutralize these invading pathogens. Among them, lymphocytes have an important characteristic, which is their ability to specifically recognize antigens, a feature not possessed by any other cell. This means that any lymphocyte function stimulated by an antigen is directed solely at that antigen.

Lymphocytes may be divided into two major populations: T and B. T -lymphocytes have a central role in regulating the immune response and for this they produce and secrete lymphokines (i.e.interleukins). On the other hand, B -lymphocytes are the only cells that produce antibodies, which are proteins that recognize and bind antigens.

Some T-lymphocytes are known as helper s (Th-lymphocytes) because they assist B-cells to produce antibodies. T -lymphocytes express a characteristic membrane molecule designated CD4. Other T -lymphocytes are known as cytotoxic (CTL) because they are capable of killing certain cells. They express a different characteristic membrane protein designated CD8.

In mice, Th-lymphocytes have been subdivided in groups which are designated Th0, Th1 and Th2, according to the lymphokines they produce. In general, Th1 lymphocytes produce lymphokines which stimulate macrophages and CTLS (IL2, IFN γ, TNF-β); Th2 lymphocytes produce lymphokines which stimulate B - lymphocytes to proliferate and to produce antibodies (IL 2, IL5, IL6, IL10, IL13); and Th0 lymphocytes produce a mixture of lymphokines and are thought to be an intermediate stage from which Th1 and Th2 lymphocytes are derived. In humans, Th1- and Th2- like lymphocytes have been demonstrated, although they seem to show a less strict division with respect to their patterns of cytokine secretion.

A third population of lymphocytes, which lack the major makers of T and B cells include the natural killer cells (NK cells), the killer cells (K cells) and the lymphokine-activated killer cells (L A K cells). NK cells can kill certain tumor cells and some virally infected cells, but unlike cytotoxic T -lymphocytes, they are not capable of recognizing a specific antigen. First, they can bore a hole in a target cell by secreting perforin molecules to form a membrane attack complex on the surface of the target. NK cells can then secrete enzymes that enter the target cell and cause it to commit suicide. As a second mechanism of destruction, Fas ligand, a protein present on NK cells can interact with Fas, another protein present on the surface of the target cell, inducing the target cell to commit suicide by ap optosis. NK cells are also able to bind to cells, which have antibody to them via their antigen -binding regions and kill them. L A K cells do not specifically recognize an antigen but they are capable of destroying a wider range of targets than NK cells.

Macrophages and dendritic cells play a critical role in initiating immune responses, helping T cells to respond to antigens.

There are several classes of antibody. The IgG class comprises most of the circulating antibodies and it has four subclasses designated IgG1, IgG2, IgG3 and IgG4. The IgM class comprises about 10 % of the circulating antibodies. These are the principal antibodies produced during the primary immunological response. The IgA class comprises most of the antibodies secreted at mucous membranes and exerts its protective effect by blocking access of the antigen to the inner body. The IgD class comprises less than 1 % of scrum antibodies and its biological role is largely unknown. The IgE class comprises antibodies that are mainly bound to the surface of most cells and basophils. These antibodies are associated with reactions that occur in individuals who are undergoing allergie reactions.

### Cytokines

Cytokines are proteins produced by cells in response to a variety of stimuli and that influence the behavior of other (target) cells. The nature of the target cells for a particular cytokine is determined by a specific receptor and the resulting effects inside the target cell are brought about by signal transduction pathways (see M.J.

Clemens, "Cytokines" , BIOS Scientific Publishers Limited, Oxford, U.K., 1991). As key mediators of the immunological defense processes, cytokines are involved in the response to exogenous and endogenous insults (invading organisms, turnors, and trauma), repair and restoration of the homeostasis. Therefore, the administration of pharmacological doses of cytokines is being used or explored in a wide variety of infectious diseases, autoimmune diseases and neoplasias.

### Granulocyte-Macrophage Colony Stimulating Factor (GM -CSF)

GM-CSF is a cytokine able to modulate the growth or differentiation of haemopoietic cells (Rasko, JEJ, Gough, NM. Granulocyte -Macrophage Colony Stimulating Factor.

In *The cytokine Handbook*. A. Thomson, Academic Press, N.Y. p.343). GM -CSF stimulates the formation of a range of myeloid colony types in semi -solid cultures "in vitro" in a dose-dependent manner (Metcalf et al. Blood 67:37, 1986), and elevates macrophages, neutrophils and eosinophils "in vivo" in mice and monkeys (Metcalft et al. Exp. Hematol. 15:1, 1987; Mayer et al. Blood 70:706, 1987). GM -CSF induce "in vitro" maturation of granulocytes, macrophages, eosinophils,megacaryocytes and dendritic cells (Metcalf, D. Proc. Natl. Acad. Sci. USA 77:5327, 1980; Metcalf, D, Burgess, A.W. J. Cell Physiol, 1 11:275, 1982; Inaba, K. et al.J. Exp. Med. 175:1157, 1992; Caux, C. et al.Nature 360:258, 1992.; Clark, E.A. et al. J. Immunol. 148:3327, 1992.) and induce "in vivo" the expansion and differentiation of dendritic cells in animals and humans (Hanada, K. et al. J. Leukoc. Biol. 60: 181, 1996; Storozynsky, E. et al. Immunology, 97: 138, 1999; Kim, J.J. et al. Hum. Gene Ther. 11: 305, 2000;

Roth, M.D., et al. Cancer Res. 60:1934, 2000; Burnham, K. et al. J. Interferon Cytokine Res. 20: 1071, 2000; Basak, S.K. et al. Blood, 99:2869, 2002; Peretz, Y. et al. Mol. Ther. 6: 407, 2002). According to these properties, the GM -CSF was explored for clinical applications. Current clinical applications of the GM -CSF are: amelioration of acute and chronic states of neutropenia (Liang, D.C. Paediatr. Drugs, 5: 673, 2003; Dale, D.C. 62: 1, 2002; Bruserud, O. et al. Eur. Cytokine Netw. 12:231, 2001; Rubenstein, E.B. Int. J. Antimicrob. Agents.16:117 , 2000), adjunctive therapy with anti-microbials in nonneutropenic states (Held, T.K., Cross, A.S. Curr. Opin. Hematol. 6:176, 1999; Root, R.K., Dale, D.C. J. Infect. Dis. 179: S342, 1999, Daifuku, R. et al. J. Antimicrob. Chemother. 32: Suppl A: 91, 1993), and enhancement of the anti -tumor or antimicrobial immunity (Schadendorf, D . Drug News Perspect. 13: 85, 2000; Demir, G. et al. Leuk. Res. 27: 1105, 2000; Jager, E. Int. J. Cancer. 106: 817, 2003; Ullenhag, G.J. et al. Clin. Cancer Res. 9: 2447, 2003; Singh, N.P. et al. Ren. Fail. 25: 255, 2003).

### Vaccines and vaccine adjuvants

Vaccines are preparations used to stimulate animals to mount an immune response against antigens included in the vaccine. Vaccines often include adjuvants, which are substances that used in combination with specific antigen produce more immunity than the antigen used alone. (Ramon, G.,1926. Procedes pour accroite la production des antitoxins. Ann. Inst. Pasteur. 40, 1 - 10).

Many kinds of compounds function as vaccine adjuvants (Edelman, R. , 2000. An overview of adjuvant use, in : Vaccine Adjuvants. Preparation Methods and Research Protocols. D.T. O' Hagan, Ed., Humana Press, Totowa, New Jersey. References cited in this article are incorporated herein as background material).

However, currently, the only adjuvants approved for use in humans are aluminum salts (Gupta, R.K. and Rost, B.E., 2000. Aluminum compounds as vaccine adjuvants in: Vaccine Adjuvants. Preparation Methods and Research Protocols. D.T. O' Hagan,

Ed., Humana Press, Totowa, New Jersey) and the oil -in-water emulsion M F 59 (Ott, G. Radhakrishman, R. Fang, J. and Hora, M., 2000. The adjuvant M F 59: A 10 - Year Perspective, in : Vaccine Adjuvants. Preparation Methods and Research Protocols. D.T. O' Hagan, Ed., Humana Press, Totowa, New Jersey).

### Immunotherapy of cancer

Multiple immunotherapeutic strategies have been developed to control cancer; they include (a) local application of a live bacterial vaccine, BCG; (b) use of cytokines; (c) active immunization; (d) passive therapy with antibodies; and (c) adoptive transfer of effector cells. All these treatments are aimed to activate the immune system so it can recognize tumoral cells as non -sclf cells (Whiteside, T.L. J. Allergy Clin. Immunol. 11:S677,2003).

### Nucleic acids as immunostimulatory compounds

Several polynucleotides have been demonstrated to have immunostimulatory properties. For example, poly (I,C) is an inducer of interferon (IFN) production, macrophage activation and NK cell activation (Talmadge, J.E., Adams, J., Phillips, H., Collins, M., Lenz, B., Schneider, M., Schlick, E., Ruffm ann, R., Wiltrout, R.H., Chirigos, M.A.1985. Immunomodulatory effects in mice of polyinosinic - polycytidylic acid complexed with poly -L:-lysine and carboxymethylcellulose. Cancer Res. 45:1058; Wiltrout, R.H., Salup, R.R., Twilley, T.A., Talmage, J.E. 1985. Immunomodulation of natural killer activity by polyribonucleotides. J. Biol. Resp. Mod. 4:512), poly (dG,dC) is mitogenic for B cells (Messina, J.P., Gilkerson, G.S., Pisetsky, D.S. 1993. The influence of DNA structure on the in vitro stimulation of murine lymphocytes by natural and synthetic polynucleotide antigens. Cell. Immunol. 147:148) and induces IFN and NK activity (Tocunaga, T., Yamamoto, S., Namba, K.1988. A synthetic single -stranded DNA, poly(dG,dC), induces interferon -α/β and -γ, augments natural killer activity, and suppresses tumor growth. Jpn. J. Cancer Res. 79:682).

Bacterial DNA has also been reported to have immunostimulatory properties. These properties include the induction of cytokines (interferon gamma (IFN γ), alpha (IFN α) and beta (IFN β)), tumor necrosis factor alpha (TNF α), interleukin 6 (IL6), 12 (IL 12) and 18 (IL 18), as well as the direct stimulation of B cells (Yamamoto, S. et al. 1988. In vitro augmentation of natural killer cell activity of interferon α/β and γ with deoxyribonucleic acid fraction from *Mycobacterium bovis* BCG. Jpn. J. Cancer Res. (Gann) 79: 866-873; Yamamoto S. et al., 1992. DNA from bacteria, but not from vertebrates, induces interferons, activates natural killer cells and inhibits tumor growth. Microbiol. Immunol. 36: 983-997; Klinman, D.M., Yi, A-K., Beaucage, S.L., Conover, J. and Krieg, A.M.,1996. CpG motifs present in bacterial DNA rapidly induce lymphocytes to secrete interleukin 6, interleukin 12 and interferon γ. Proc. Natl. Acad. Sci. USA 93, 2879-2883. Halpern, M. D., et al. 1996. Bacterial DNA induces murine interferon -γ production by stimulation of interleukin -12 and tumor necrosis factor -α. Cell. Immunol. 167: 72-78. Sparwasser, T. et al., 1997. Macrophages sense pathogens via DNA motifs: induc tion of tumor necrosis factor -α - mediated shock. Eur. J. Immunol. 27: 1671-1679; Krieg, A.M. ct al., 1995. CpG motifs in bacterial DNA trigger direct B -cell activation. Nature 374: 345-349).

In contrast, it has been reported that mammalian DNA has no sig nificant immune effects (Pisetsky, D.S. 1996. The immunologic properties of DNA. J. Immunol. 156: 421-423; Messina et al. 1991. Stimulation of in vitro murine lymphocyte proliferation by bacterial DNA. J. Immunol. 147: 1759).

Synthetic DNA has also been reported to be immunostimulatory if it contains unmethylated CpG motifs. (Yamamoto, S et al.; 1992. Unique palindromic sequences in synthetic oligonucleotides are required to induce INF and augment INF -mediated natural killer activity. J. Immunol. 148: 4072-4076; Ballas, Z.K., et al.; 1996. Induction of NK activity in murine and human cells by CpG motifs in oligodeoxynucleotides and bacterial DNA. J. Immunol. 157: 1840-1845; Hartmann, G., Krieg, A.M. 2000. Mechanism and function of a newly identified CpG DNA motif in human primary B cells. J. Immunol. 164:944; Hartmann, G., Weeratna, R.D., Ballas, Z.K., Payette, P., Blackwell, S., Suparto, I., Rasmussen, W.L., Waldschmidt, M., Sajuthi, D., Purcell, R.H., Davis, H.L., Krieg, A.M. 2000. Delineation of a CpG phosphorothioate oligodeoxynucleotide for activating primate immune responses in vitro and in vivo. J. Immunol. 164:1617; Verthelyi, D., Ishii, K.J., Gursel, M., Takeshita, F., Klinman, D.M. 2001. Human peripheric blood cells differentially rccognizc and respond to two distinct CpG motifs. J. Immunol. 166:2372). However, one oligonucleotide containing phosphorothioate bonds that lack CpG motifs has been found to have some immunostimulatory activity on human B cells (Liang, H., Nishioka, Y., Reich, C.F., Pisetsk y, D.S., Lipsky, P.E. 1996. Activation of human B cells by phosphorothioate oligonucleotides. J. Clin. Invest. 98:1119). This particular non-CpG oligonucleotide containing phosphorothioate bonds is a po ly-T chain, 20 nucleotides long. Also, Vollmer et al (Vollmer J, Janosch A, Laucht M, Ballas ZK, Schetter C, Krieg AM. Highly immunostimulatory CpG -free oligodeoxynucleotides for activation of human leukocytes. Antisense Nucleic Acid Drug Dev.12:165 -175, 2002) reported immunostimulation by phosphorothioate p oly-T ODNs. These authors pointed out that poly-T ODNs are only active as phosphorothioate ODNs and have much lower activity than CpG ODNs. In a later study, it was reported that non -CpG with PyNTTTTGT motifs have strong immunomostimulatory properties, directly acting on B cells and plasmacytoid dendritic cells (Elias, F. et al. J. Immunol. 171:3697,2003).

It is now disclosed that oligonucleotides having the following general sequence: TTTTTTTTTTTTCATTTTGTTTXPu wherein X is G, A or T and Pu is A or G, have the ability to stimulate white cells of the human immune system in order to secrete high amounts of GM -CSF.

Therefore, these oligonucleotides can be administered to subjects to treat white blood cell deficiencies or used as adjuvants, in conjunction with a vaccine, to boost the immune system in order to have a better response to the vaccine or administered to subjects to increase their responsiveness to tumors.

### SUMMARY OF THE INVENTION

Oligonucleotides having the following general sequence: TTTTTTTTTT TTCATTTTGTTTXPu Wherein X is G, A or T and Pu is A or G, that have the ability to stimulate white cells of the human immune system in order to produce high amounts of GM -CSF are disclosed.

The oligonucleotides of this invention are useful as adjuvants in a vaccine formulation comprising one or more antigens. In embodiments of this aspect, the vaccine formulation can be liquid or lyophilized in dosage form. Many dosage forms are known in the art and can be applied herein. In embodiments of this aspect, the oligonucleotides of this invention are present in the composition at a dose of from about 10 to 10,000 µg per dose. In these preparations, the oligonucleotides of this invention may be combined with other immunostimulatory compounds. Examples of well-known immunostimulatory compounds are: α-interferon, β-interferon, γ-interferon, interleukin 2 (IL2), interleukin 12 (IL12) and immunostimulatory oligonucleotides.

In preferred embodiments, the antigenic component of the vaccine is one or more antigens, either natural or recombinant, of viruses like: Human immunodeficiency viruses (either HIV-1 or HIV-2), polio viruses, hepatitis A virus, human coxsackie viruses, rhinoviruses, echoviruses, equine encephalitis viruses, rubella viruses, dengue viruses, encephalitis viruses, yellow fever viruses, coronaviruses, vesicular stomatitis viruses, rabies viruses, ebola viruses, parainfluenza viruses, mumps virus, measles virus, respiratory syncytial virus, influenza viruses, Hanta viruses, bunga viruses, hemorrhagie fever viruses, reoviruses, orbiviruses, rotaviruses, Hepatitis B virus, parvoviruses, papilloma viruses, polyoma viruses, adenoviruses, herpes simplex virus (HSV) 1 and 2, varicella zoster virus, cytomegalovirus (CMV), variola viruses, vaccinia viruses, poxviruses, African swine fever virus, the unclassified agent of delta hepatitis, the agents of non -A, non-B hepatitis; or one or more antigens of infectious bacteria like: *Helicobacter pylori, Borrelia burgdorferi, Legionella pneumophila, Mycobacterium tuberculosis, Mycobacterium bovis (BCG), Mycobacterium avium, Mycobacterium intracellulare, Staphylococcus aureus, Neisseria gonorrhoeae, Neisseria meningitidis, Listeria monocytogenes, Streptococcus pyogenes, Streptococcus pneumoniae, Haemophilus influenzae, Moraxella catharralis, Klebsiella pneumoniae, Bacillus anthracis, Corynebacterium diphtheriae, Clostridium perfringers, Clostridium tetani, Enterobacter aerogenes, Klebsiella pneumoniae, Pasturella multocida, and Treponema pallidum ;* or one or more antigens of infectious fungi like: *Cryptococcus neoformans, Histoplasma capsulatum, Coccidioides immitis, Blastomyces dermatitidis, Candida albicans;* or one or more antigens of infectious protists like: *Plasmodium falciparum, Trypanosoma cruzi, Leishmania donovani* and *Toxoplasma gondii* or one or more antigens of human tumoral cells.

In embodiments of this aspect, the antigen, together with one or more of the oligonucleotides of this invention, is administered simultaneously either locally (by oral, rectal, intranasal or transdermal route) or systemically (by intradermic or intramuscular injection).

An aspect of this invention is a method of vaccination of a person. The person can be vaccinated *prophylactically* or *therapeutically*.

A *prophylactic* vaccine is designed to elicit protection from a disease caused by an infectious agent through the induction of specific immunity.

A *therapeutic* vaccine is designed to induce remission of an illness (i.e. a tumor and metastasis or an illness associated with an infectious agent such as the human immunodeficiency virus).

The method of vaccination includes administering one or more of the oligonucleotides of this invention and one or more antigens - that is, the vaccine can be designed against one disease target or a combination of disease targets.

Another aspect of this invention is a method of treatment of a person with a tumoral disease with one or more of the oligonucleotides of this invention to stimulate his/her endogenous immune response. Examples of tumoral disease are: Chronic Myelogenous Leukemia, Precursor B -lymphoblastic lymphoma, B-cell chronic lymphocytic leukaemia, Lymphoplasmacytic lymphoma, Mantle cell lymphoma, Follicle center lymphoma, (follicular and diffuse), Marginal zone -B lymphoma, Extranodal lymphoma, Nodal marginal zone B-cell lymphoma, Splenic marginal zone B-cell lymphoma, Hairy cell leukaemia, Plasmocytoma, Diffuse large B -cell lymphoma, Burkitt's lymphoma, High grade B -cell lymphoma, Burkitt like, Melanoma, Kaposi's Sarcoma, Multiple Myeloma, Renal Cell Carcinoma, Bladder Cancer, Lung Cancer, Skin Cancer, Breast Cancer, Colon Cancer and Uterus Cancer.

In embodiments of this aspect, one or more of the oligonucleotides of this invention are present in a pharmaceutical formulation that can be liquid or lyophilized in dosage form. Many dosage forms are known in the art and can be applied herein. In embodiments of this aspect, one or more of the oligonucleotides of this invention are present in the composition at a dose of from about 10 to 10,000 µg per dose. In these preparations, one or more of the oligonucleotides of this invention may be combined with other immunostimulatory compounds. Examples of well -known immunostimulants are: α-interferon, β-interferon, γ-interferon, interleukin 2 (IL2), interleukin 12 (IL12), CpG oligonucleotides, PyNTTTTGT oligonucleotides and *Mycobacterium bovis* BCG cells. Also, one or more of the oligonucleotides of this invention may be combined with an antiinfective or anticancer drug, or with a surgical procedure. In all these cases, the oligonucleotides of this invention may be administered either before or after the alternative treatment, or simultaneously.

Another aspect of this invention is that a person with a tumoral disease could be treated by contacting dendritic cells from the subject, with one or more of the oligonucleotides of this invention "ex vivo" and re -administering the activated cells to the subject. In embodiments of this aspect, one or more of the oligonucleotides of this invention are present in the incubation media in a concentration of about 0.10 to 100 µg per ml.

### BRIEF DESCRIPTION OF THE FIGURES

Fig 1 is a graph plotting the secretion of GM -CSF by human PBMC incubated with the indicated phosphorothioate ODNs. Data represent the mean and standard deviation of five independent assays.
Fig 2 is a graph plotting the secretion of GM -CSF by PBMC incubated with the phosphorothioate ODN IMT700 (SEQ. ID N° 8) in the presence of different amounts of IFN alpha. Data represent the mean and standard deviation of five independent assays.
Fig 3 is a graph plotting the secretion of GM -CSF by PBMC incubated with the indicated phosphorothioate ODNs or mix of ODNs. Data represent the mean and standard deviation of five independent assays.
Fig 4 shows the inhibition by chloroquine of the induction of immunoproliferation (a), induction of secretion of IL6 (b) and induction of CD40 (c) caused by the oligonucleotide IMT504 (SEQ. ID N° 2) acting on B celts in human PBMC.
Fig 5 shows the lack of inhibition by chloroquine of the secretion of GM-CSF induced by the oligonucleotide ODN IMT700 (SEQ. ID N° 8) of this invention. Chloroquine was used at a final concentration of 5 µg/ml.
Fig 6 shows the anti-hepatitis B antibody titers in rats vaccinated with hepatitis B antigen plus alumina or hepatitis B antigen plus alumina and ODN IMT700 (SEQ. ID N° 8).
Fig 7 shows the IgG2b/IgG relationship in rats vaccinated with hepatitis B antigen plus alumina or hepatitis B antigen plus alumina and ODN IMT700 (SEQ. ID N° 8).

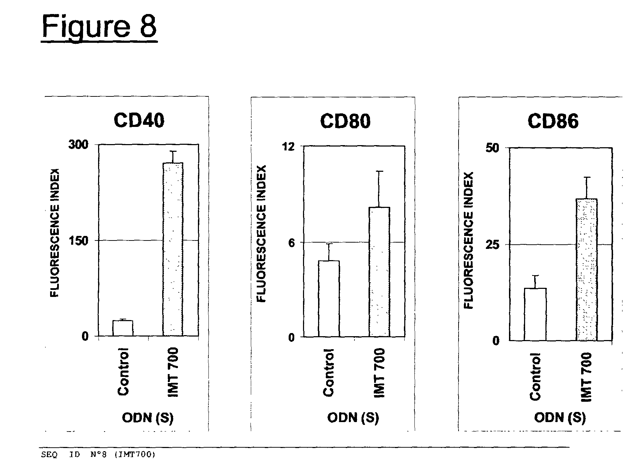

Fig 8 shows the induction of CD40, CD80 and CD86 in CD 19+ (B cells) of a patient suffering B cell chronic lymphocytic leukemia (CLL) by the ODN IMT700 (SEQ. ID N° 8) of this invention. PMBC of the patient were cultured for 24h with the ODN IMT700 and then stained with fluorescent anti-CD19/anti-CD40 (a), anti-CD19/anti-CD80 (b), or anti-CD19/anti-CD86 (c). Flow-cytometric results are presented as histograms corresponding to CD19+ cells. Open histograms correspond to cells cultured in absence of ODN and shaded histograms to cells cultured in presence of the ODN. ODN (S) means phosphorothioate ODN.

### DETAILED DESCRIPTION OF THE INVENTION

### Definitions

- As used herein, the term "oligonucleotide" or "oligo" means multiple nucleotides, i.e. molecules comprising a sugar (e.g. ribose or deoxy ribose) linked to a phosphate group and to an exchangeable organic base, which is either a substituted pyrimidine (e.g. cytosine (C), thymine (T) or uracil (U)) or a substituted purine (e.g. adenine (A) or guanine (G)). The term "oligonucleotide" as used herein refers to both oligoribonucleotides (ORNs) and oligodeoxyribonucleotides (ODNs). The term "oligonucleotide" also includes oligonucleosides (i.e. an oligonucleotide minus the phosphate) and any other organic base containing polymer. Oligonucleotides can be obtained from existing nucleic acid sources (e.g. genomic or cDNA), but are preferably synthetic (e.g. produced by oligonucleotide synthesis).
- An "oligonucleotide" refers to multiple nucleotides linked by phosphodiester bonds.
- An "immunostimulatory oligonucleotide" refers to an oligonucleotide which stimulates (i.e. has a mitogenic effect on, induces, increases or decreases cytokine expression in a cell of the immune system (i.e. a lymphocyte, a macrophage) in a statistically significant manner.
- A "CpG" refers to a cytosine -guanine dinucleotide.
- A "CpG oligonucleotide" refers to an oligonucleotide which stimulates a cell of the immune system and its immunostimulatory activity critically depends on the presence of at least one CpG in its sequence.
- A "non-CpG oligonucleotide" refers to an oligonucleotide that does not have a CpG in its sequence.
- An oligonucleotide that induces "high" amounts of GM -CSF is one that under the conditions described in the example 1 induces the secretion of more than 200 pg of GM-CSF/ml of culture media.
- As used herein, the term "treating" refers to a process by which the symptoms of a disease, and more particularly, infectious diseases or tumoral diseases are ameliorated or completely eliminated.
- As used herein, the term "preventing" refers to a process by which a disease, and, more particularly, infectious diseases or tumoral diseases are obstructed or delayed.
- In a preferred embodiment, the immunostimulatory oligonucleotides of the invention are advantageously modified into stabilized oligonucleotides. Such stabilized immunostimulatory oligonucleotide may be particularly useful to obtain a prolonged immunostimulation. As used herein, a "stabilized oligonucleotide" refers to an oligonucleotide that is relatively resistant to in vivo degradation (e.g. via an exo- or endo-nuclease). Preferred stabilized oligonucleotides of the present invention comprise a phosphate backbone modification. More particularly, the phosphate backbone modification is preferably a 5' inter-nucleotide linkage modification, for instance, at the first two nucleotides of the 5' end of the oligonucleotide of the invention. Furthermore, the phosphate backbone modification may be a 3' inter-nucleotide linkage modification. In such a case, the modification may occur, for instance, at the last two nucleotides of the 3' end of the oligonucleotide of the invention. Even more preferably, the immunostimulatory oligonucleotide of the invention may be stably modified so as to comprise a phosphorothioate-linked nucleotide (i.e. at least one of the phosphate oxygen molecules is replaced by sulfur). In the most preferred embodiment, most of, if not all, the nucleotides of the immunostimulatory oligonucleotides of the invention comprise a phosphorothioate -linked nucleotide.
- Other stabilized oligonucleotides may alternatively include : nonionic DNA aanalogs, such as alkyl- and aryl- phosphonates (in which the charged phosphonate oxygen is replaced by an alkyl or aryl group), phosphodiester and alkylphosphotriesters, in which the charged oxygen moiety is alkylated.
Oligonucleotides which contain a diol, such as tetraethyleneglycol or hexaethyleneglycol, at either or both termini have also been shown to be substantially resistant to nuclease degradation.

### Description

The present invention provides methods to augment the immune response of humans by adding one or more of the oligonucleotides of this invention to vaccines, or performing a treatment based on the administration of one or more of the oligonucleotides of this invention to a person with a tumoral disease, or contacting "ex vivo" white blood cells obtained from a person with a tumoral disease with one or more of the oligonucleotides of this invention and re -administering these activated white blood cells to the same person.

Vaccine compositions containing one or more of the oligonucleotides of this invention can present antigens directly (i.e. in the form of a defined protein or polysaccharide) or as a part of a complex biological entity (i.e. complete viruses; complete bacterial cells; bacterial membranes or artificial conjugates like polysaccharide-protein conjugates). These antigens can be combined in multiple vaccines.

A vaccine composition including at least one antigen is formulated to include one or more of the oligonucleotides of this invention.

For example, the antigen can be *Moraxella catharralis* killed cells, subcellular fractions of these cells, or *Hepatitis B virus* surface antigen, either natural or produced by means of the DNA recombinant technology.

One or more of the oligonucleotides of this invention may be formulated alone or together with one or more antigens in a pharmaceutical composition, which may also include carriers, thickeners, diluents, buffers, preservatives, surface active agents, anti-microbial agents, anti-inflammatory agents, anesthetics and the like.

The formulation can be liquid or lyophilized. The pharmaceutical composition may be administered in a number of ways depending on whether local or systemic treatment is desired, and on the area to be treated. Administration may be done topically, orally, by inhalation or parenterally. Formulation for topical administration may include ointments, lotions, creams, gels, drops, suppositories, sprays, liquids and powders. Pharmaceutical carriers, aqueous, powder or oily bases, thickeners and the like may be necessary or desirable. Formulations for oral administration include powders or granules, suspensions or solutions in water or non -aqueous media, capsules, tablets and the like. Thickeners, flavorings, diluents, emulsifiers and the like may be necessary or desirable. Formulations for parenteral administration include sterile aqueous solutions, which may also contain buffers, diluents and other additives. A vaccine containing one or more antigens and one or more of the oligonucleotides of this invention can be formulated and used for prophylactic or therapeutic purposes.

Common antigens used in viral prophylactic vaccines are from *Hepatitis B virus, Rabies virus, Polio virus, Hepatitis A virus* and *Influenza virus.* Common antigens used in bacterial prophylactic vaccines are from *Streptococcus pneumoniae, Haemophilus influenzae, Moraxella catharralis, Klebsiella pneumoniae and Mycobacterium bovis (BCG)*. Common antigens used in therapeutic vaccines are from *Papilloma virus, HIV virus* and melanoma cells.

A further refinement of a vaccine formulation is to incorporate one or more of the oligonucleotides of this invention as adjuvant/s and the antigen/s into a delivery vehicle to provide a delayed release of the active compounds of the vaccine over time. This can be accomplished by various means known in the art. Examples of these means are encapsulation into Poly (lactide -coglicolide) micro particles (Kersten, G.F.A. and Gander, B. 1996. Biodegradable Micro Spheres as vehicles for antigens, in: S.H.E. Kaufmann, ed. Concepts in Vaccine Development. Walter de Gruyter. Berlin-New York), liposomes (Gregoriadis, G. et al. 2000. Liposomes as Immunological Adjuvants and Vaccine Carriers, in: S.H.E. Kaufmann, ed. Concepts in Vaccine Development. Walter de Gruyter. Berlin-New York) and poly (methyl methacrylate) nanoparticles (Kreuter, J. 2000. Poly (Methyl Methacrylate) nanoparticles as vaccine adjuvants, in: S.H.E. Kaufmann, ed. Concepts in Vaccine Development. Walter de Gruyter. Berlin-New York).

Another refinement of the vaccine formulation is to conjugate the antigen/s and one or more of the oligonucleotides of this invention, by chemical means (Mier W, Eritja R, Mohammed A, Haberkorn U, Eiscnhut M.2000. Preparation an d evaluation of tumor-targeting peptide-oligonucleotide conjugates. Bioconjug. Chem. 11:855).

Many vaccine formulations are known in the art and can be used by substituting one or more of the oligonucleotides of this invention for the adjuvant previously known or by simply adding one or more of the oligonucleotides of this invention to the original formulation.

Based on their immunostimulatory properties, one or more of the oligonucleotides of this invention can also be administered to a subject in vivo to treat a tumoral disease. Examples of common tumoral diseases are: Chronic Myelogenous Leukemia, Melanoma, Kaposi's Sarcoma, Multiple Myeloma, Renal Cell Carcinoma, Bladder Cancer, Lung Cancer, Skin Cancer, Breast Cancer, Colon Cancer and Uterus Cancer. Examples of common immunological disorders are: Allergy, Severe Combined Immunodeficiency, Chronic Granulomatous disease, and Acquired Immunodeficiency Disease.

The pharmaceutical composition for these treatments may include one or more of the oligonucleotides of this invention together with carriers, thickeners, diluents, buffers, preservatives, surface active agents, anti -microbial agents, anti -inflammatory agents, anesthetics and the like. The formulation can be liquid or lyophilized.

The pharmaceutical composition may be administered in a number of ways depending on whether local or systemic treatment is desired, and on the area to be treated. Administration may be done topically, orally, by inhalation or parenterally. Formulation for topical administration may include ointments, lotions, creams, gels, drops, suppositories, sprays, liquids and powders. Pharmaceutical carriers, aqueous, powder or oily bases, thickeners and the like may be necessary or desirable. Formulations for oral administration include powders or granules, suspensions or solutions in water or non -aqueous media, capsules, tablets and the like. Thickeners, flavorings, diluents, emulsifiers and the like may be necessary or desirable.

Formulations for parenteral administration include sterile aqueous solutions, which may also contain buffers, diluents and other additives. Alternatively, one or more of the oligonucleotides of this invention can be contacted "*ex vivo*" with white blood cells obtained from a subject having a tumoral disease or an immune system deficiency and activated cells can then be reintroduced in the subject.

### EXAMPLE 1

### Materials and Methods

The following materials and methods were generally used throughout the examples.

### 1) Oligonucleotides

Oligonucleotides having phosphorothioate internucleotide linkages were purchased, purified by high-pressure liquid chromatography (HPLC), from Oligos Etc (Bethel, Maine). Oligonucleotides were suspended in depyrogenated water and kept at -20° C until used.

### 2) Antibodies

Antibodies used in assays were purchased from Serotec (Raleigh, NC).

### 3) Peripheral mononuclear blood leukocytes (PBMC)

PBMC were obtained as previously described (Ballas, Z. K. et al., (1990) J. Allergy

Clin. Immunol. 85:453; Ballas, Z. K. and W. Rasmussen (1990) J. Immunol. 145:1039; Ballas, Z. K. and W. Rasmussen (1993) J. Immunol. 150;17).

### 4) GM-CSF secretion assays

PBMC were prepared from whole blood by standard centrifugation over ficoll hypaque. Cells (1.times.10.sup.6 /ml) were cultured in RPMI -1640 media supplemented with 0.5% (v/v) heat inactivated fetal calf serum, 400 IU/ml human recombinant IL2 (Proleukin®, Chiron Corporation), 1.5 .mu.M L -glutamine and gentamicin, in 96 well microtiter plates with oligodeoxynucleotides (6 µg /ml) for 72 h before supernatant harvest and assay by ELISA. For ELISA tests, ninety -six-well microtiter plates were coated with anti -GM-CSF antibodies and blocked with PBS - 5% BSA. GM-CSF in cultures was detected colorimetrically using biotin -labeled antibodies followed by peroxid ase-conjugated strcpto-avidin and then peroxidase-specific colorimetric substrate. Standard curves were generated to quantitate ELISA results using known amounts of recombinant GM -CSF. The detection limit of the assays was 10 pg/ml. All assays were performed in duplicate.

### 5) Flow cytometry

Staining of surface antigens was performed as described (J. Fló and E. Massouh, Age-related changes of native and memory CD4 rat lymphocyte subsets in mucosal and systemic lymphoid organs. Developmental and comparative Immunology 21: 443-453, 1997). CD19 (Clone LT19) and CD86 (Clone BU63) antibodies were purchased from Serotec (Raleigh, NC).

Flow cytometric data of 10,000 cells/sample were acquired on a FACScan (Becton

Dickinson Immunocytomctry Systems, San Josc, CA). Data were analyzed using the computer program Win MDI (Windows Multiple Document Interface; Flow Cytometry Application. Joseph Trotter, Copyright 1993 -1998) Version 2.8.

### 6) Animals

Female Sprague Dawley 8-12 weeks-old rats were obtained from FUCAL Laboratories (Buenos Aires, Argentina) and housed in a facility at the School of Natural Sciences, University of Buenos Aires, Buenos Aires, Argentina.

Animal care and use were according to institutional guidelines.

### 7) Vaccines

Study products consisted in a recombinant surface antigen of the Hepatitis B virus (HBsAg) (Pablo Cassará SRL, Buenos Aires, Argentina) alone or in combination with either alum or in combination with the ODN IMT700 (Seq. ID N° 8); or in combination with both alum and IMT700. Study products were stored at -70 °C, and were diluted with sterile buffered saline to achieve the desired concentration.

### 8) Immunization of rats

Immunization of rats was conducted on 8 - to 12- wk -old female animals. Each rat received a single i.m. vaccine injection in the tibialis anterior muscles. The control groups received the vaccine alone while the treated groups received the vaccine with the addition of IMT700. Four weeks later, rats were bled and total IgG, IgG1, IgG2a IgG2c and IgG2b specific for each antigen were determined by ELISA using HBsAg - coated plates.

### 9) Serum antibody determinations in rats

Sera were recovered from rats 4 weeks after immunization. Specific anti -HBsAg antibody titers were determined by end -point dilution ELISA assays. In brief, ELISA plates (Maxisorb, Nunc) were coated overnight with 3 µg/ml recombinant HBsAg. Residual protein-binding sites were blocked with carbonate -bicarbonate buffer containing 8% non-fat milk. Samples were diluted 1/200 with 0.3% Tween 20 in phosphate buffered saline (PBS). Samples were further serially diluted, and plates incubated for 2 h at 37°C. Antibody subclasses were evaluated using MAbs against IgG1, IgG2a, IgG2b or IgG2c (Serotec, Raleigh , NC) followed by horseradish peroxidase (HRP)- conjugated goat anti mouse IgG. Finally, O-phenylendiamine dichloride solution (1mg/ml) was finally added to the wells. End -point titers were defined as the highest serum dilution that resulted in an absorbance value three times greater than that of non-immune serum with a cut-off value of 0.15.

### 10) Immunization of rats and sample collection

Eight to twelve week-old female Sprague Dawley rats received a single i.m. injection into the tibialis anterior muscle of a vaccine preparation containing 1, 3 or 9 µg of HBsAg in saline in a total volume of 100 µl. Animals in control groups received HBsAg with or without alum. Animals in experimental groups received HBsAg with or without alum plus 10, 50 or 250 µg of IMT700. Four weeks later rats were bled and IgG, IgG1, IgG2a, IgG2b and IgG2c titers were determined.

### 11) Statistical analysis

Statistical significance of differences was evaluated by Student's *t* test.

### EXAMPLE 2

### Search of oligonucleotides with capacity for GM -CSF induction

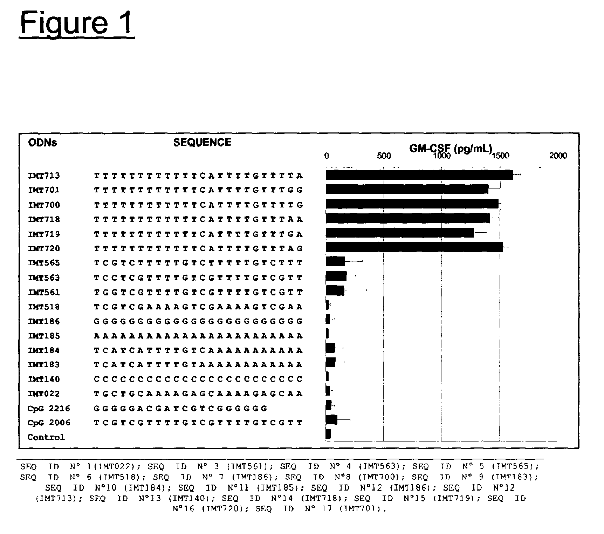

During a screening of properties associated to the immune system involving more than three hundred oligonucleotides (ODNs), with or without CpG or PyNTTITGT immunostimulatory motifs, it was found that some of them were able to significantly induce the secretion of GM -CSF by human PBMC. This fact was surprising because, even though immunostimulatory oligonucleotides have been extensively studied, they had never been reported to be able to induce the secretion of high amounts of GM - CSF (> 200 pg/ml of culture media) acting on human cells (see for example Krieg AM. *Annu. Rev. Immunol.* 20:709-760, 2002). Fig. 1 shows that none of the ODNs that were able to induce the secretion of high amounts of GM -CSF have CpG dinucleotides in its sequence and that those that were able to induce the secretion of high amounts of this cytokine have a large content of Ts and a CATTTTGT motif located from the central part of the molecule towards the 3' end. On the other hand, these oligonucleotides have in its 3' end G or A . The experiments described in Fig.1 were performed in the presence of 400 International Units (IU) of human recombinant IL2 in the culture media, a condition that increases the total amount of GM-CSF secreted by human PBMC and gives reproducibility to the results. Therefore, even though in vivo IL2 could be produced under several circumstances, the combination of one or more of the ODNs of this invention with IL2, in an appropriate concentration, may be desirable in some of the clinical applications of the ODNs of this invention.

### EXAMPLE 3

### Interferon alpha (IFN alpha) and ODNs that induce the production of Interferon alpha are inhibitors of the GM -CSF secretion induced by the ODNs of this invention

As shown in example 2, ODNs that have a CpG dinucleotide in its sequence are poor or null inducers of the secretion of GM-CSF by human PBMC. A major difference between CpG and non -CpG oligonucleotides is that the former ones are inducers of IFN alpha acting on plasmacytoid dendritic cells while the latter are not (Krieg AM.

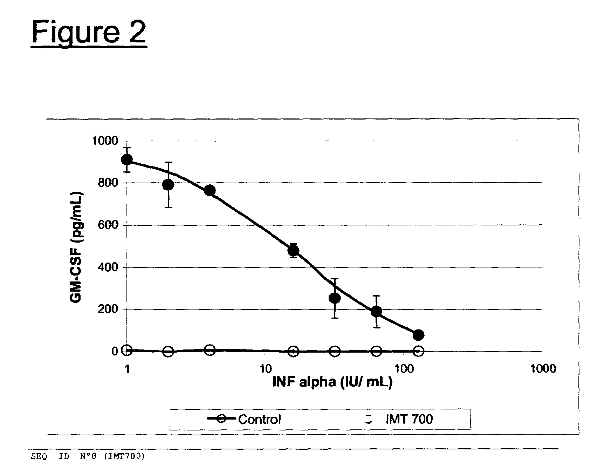

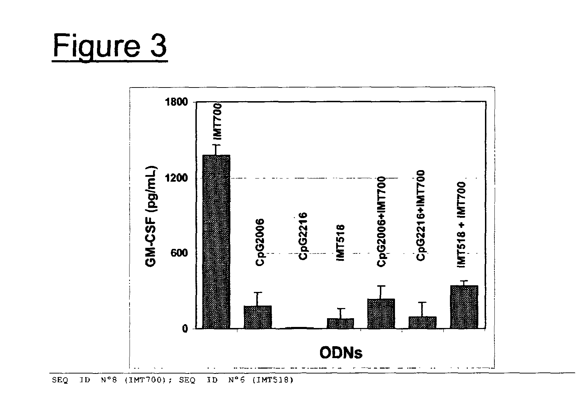

*Annu. Rev. Immunol.* 20:709-760, 2002; Elias, F. J. Immunol. 171:3697 -3704, 2003). Therefore it was important to investigate whether IFN alpha was an inhibitor of the GM-CSF secretion induced by the ODNs of this invention. Fig. 2 shows that in fact, IFN alpha is an inhibitor of the GM -CSF secretion induced by the ODNs of this invention in a concentration as low as 30 IU/ml (about 100 pg/ml). On the other hand, using a mix of CpG ODNs and ODNs of this invention it was observed that the secretion of GM-CSF by PMBC was inhibited (Fig. 3). This indica tes that the CpG ODNs inhibit the secretion of GM -CSF by acting, most probably, on plasmacytoid dendritic cells inducing the secretion of IFN alpha.

### EXAMPLE 4

### The mechanism of induction of GM -CSF by the oligonucleotides of this invention is different of the mechanism of induction of the other imunostimulatory activities previously described for immunostimulatory oligonucleotides

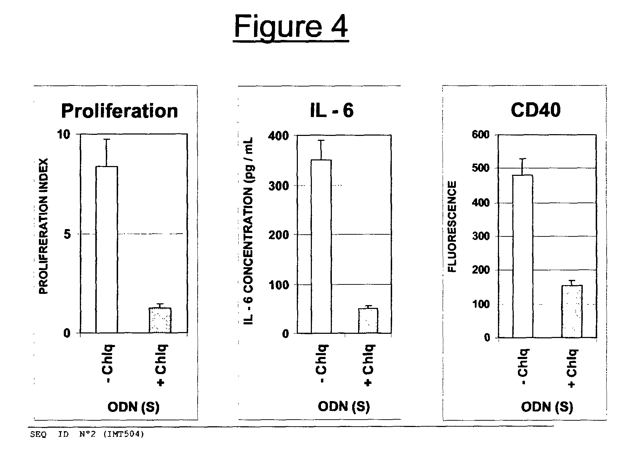

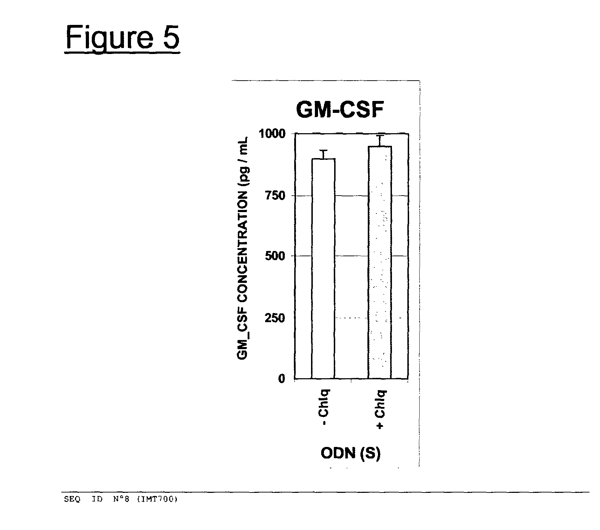

Chloroquine (Chlq) is a powerful inhibitor of the activity of CpG oligonucleotides (Krieg AM. *Annu. Rev. Immunol*. 20:709-760, 2002). On the other hand, Chloroquine was also able to inhibit the immunostimulatory activity of the (non - CpG) PyNTTTTTGT oligonucleotides as illustrated by the inhibition of the immunoproliferation, induction of the secretion of IL6 and induction of the costimulatory surface molecule CD40 in human PBMC stimulated with the prototype of the PyNTTTTGT oligonucleotides IMT504 (- SEQ ID N° 2- Fig.4 a, b and c). In contrast, the secretion of GM -CSF induced by the oligonucleotides of this invention, acting on human PBMC, was not inhibited by Chloroquine (Fig.5). *These results clearly indicate that the mechanism of induction of the secretion of GM -CSF is different from the mechanism of induction of the immunostimulatory activity of an oligonucleotide* as measured by standard markers of immunostimulation. *Therefore, induction of high levels of GM-CSF secretion in human PBMC by the oligonucleotides of this invention could have never been anticipated by anyone skilled in the art as an obvious extension of previously reveled immunostimulatory activities, different from GM-CSF secretion, present in these oligonucleotides*.

### EXAMPLE 5

### Vaccination of Rats with a recombinant hepatitis B surface antigen increases the total specific antibody title and the Th1 type of immune response

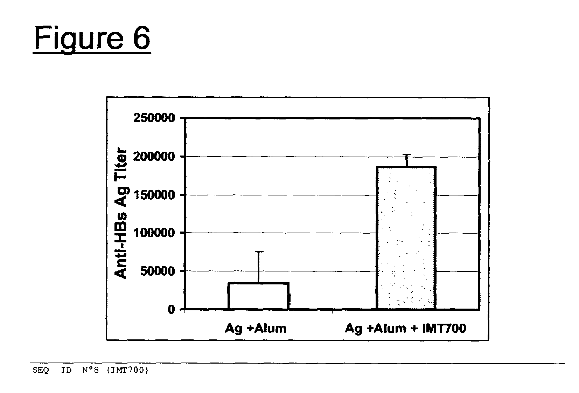

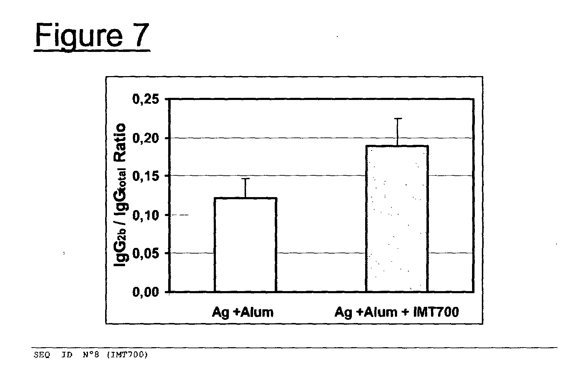

Fig. 6 shows the effect of inoculation of rats with a vaccine formulation that includes recombinant Hepatitis B surface antigen (rHBsAg) and alumina in presence or absence of one of the ODNs of this invention (IMT700, SEQ. ID N° 8) as adjuvant. As can be observed, the total IgG is about 4 times larger, in average, in rats injected with antigen plus alumina plus IMT700 than in rats injected with antigen plus alumina. On the other hand, Fig. 7 shows that IgG2b is about 7 times larger, in average, in rats injected with antigen plus alumina plus ODN IMT700 than in rats injected with antigen plus alumina indicating a shift from a predominantly Th2 type of response induced by the vaccine alone to an equilibrated Th1/ Th2 type of response induced by the vaccine plus IMT700.

### EXAMPLE 6

### Vaccination of humans

In particular, humans can be vaccinated against hepatitis B by administration of a vaccine formulation that includes recombinant Hepatitis B surface antigen (rHBsAg) and alumina and one or more of the oligonucleotides of this invention as adjuvants.

The amount of rHBsAg in each dose, and the administration schedule, can vary according to age. For example, for humans from about birth to about 12 years old, a three-dose schedule of about 2.5 µg to about 5 µg of rHBsAg can be administered at 0, 1-3 months later and 4-18 months later, preferably at 0, 2 and 6 months. One or more of the oligonucleotides of this invention can be present in the formulation from about 10 µg to about 1,000 µg per dose.

For humans from about 12 to about 60 years old, a three-dose schedule of about 5 µg to about 40 µg of rHBsAg can be administered at 0, 1 -3 months later and 4-18 months later, preferably at 0, 2 and 6 months. One or more of the oligonucleotides of this invention can be present in the formulation from about 10 µg to about 1,000 µg per dose.

### EXAMPLE 7

### Stimulation of the expression of co -stimulatory molecules in malignant B - lymphocytes by ODN IMT700

The stimulation of malignant B lymphocytes extracted from blood of patien ts suffering from chronic lymphocytic leukemia (CLL) by phosphorothioatc non -CpG oligonuclcotides of this invention was examined by flow cytometric (FACS) analysis. The results of a typical FACS analysis are shown in Fig. 7.

As can be seen, the phosphorothioate non-CpG oligonucleotide IMT700 of this invention is able to stimulate the expression of CD40, CD80 and CD86 co - stimulatory molecules on the surface of malignant B -lymphocytes.

### EXAMPLE 8

### Treatment of subjets with tumoral disease

Pharmaceutical formulations containing one or more of the immunostimulatory oligonucleotides of this invention can be used to treat subjects against a variety of tumoral diseases.

In particular, humans with B-cell leukemia can be treated by administration of a pharmaceutical formulation containing the oligonucleotide of this invention as the active component. The amount of oligonucleotide in each dose, and the administration schedule, can vary appropriately with the corporal mass of the subject and with the stage in tumor progression. For example, for humans of about 70 Kg having an advanced B -cell leukemia, a dose of about 60 mg of the oligonucleotide of this invention can be administered 3 times per week for about 4 weeks. This treatment could then be periodically repeated as necessary.

### RELEVANT REFERENCES

Patent documents
EP 0468520
US 5,663,153
US 5,723,335
US 6,090,791
US 6,194,388
US 6,207,646
US 6,239,116
US 6,406,705
US 6,426,336
US 6,429,199
US 6,498,148
US 6,514,948
US 6,544,518
US 20010044416
US 20020156033
US 20020165178
US 20020198165
US 20030050268
WO 9425588
WO 9962923
WO 95/026204
WO 96/002555
WO 98/040100
WO 98/052962
WO 99/033488
WO 99/056755
WO 00/014217
WO 00/067023
WO 00/067787
WO 00/075304
WO 00/062802
WO 01/000231
WO 01/000232
WO 01/022972
WO 01/055370
WO 01/002007
WO 01/007055
WO 01/022972
WO 01/022990
WO 01/048018
WO 01/055341
WO 011062909
WO 01/062910
WO 01/083503
WO 01/093905
WO 02/026757
WO 02/056909
WO 02/069369
WO 02/095027
WO 02/102825

### Other references

Ahmad-Nejad P et al., Bacte rial CpG-DNA and lipopolysaccharides activate Toll -like receptors at distinct cellular compartments. *Eur. J. Immunol*. 32:1958-1968, (2002).

Angier N. Microbe DNA Seen as Alien By Immune System, *New York Times,* 11, (1995).

Auf G et al., CpG-oligodeoxynucleotide rejection of a neuroblastoma in A/J mice does not induce a paraneoplastic disease. *Neurosci. Lett.* 327:189-192, (2002).

Ayash-Rashkovsky M et al., Generation of Th1 immune responses to inactivated, gp120-depleted HIV-1 in mice with a dominant Th2 bias ed immune profile via immunostimulatory oligonucleotides -relevance to AIDS vaccines in developing countries. *Vaccine.* 20:2684-2692, (2002).

Azad RF et al., Antiviral Activity of a Phosphorothioate Oligonucleotide Complementary to RNA of the Human Cytomega lovirus Major Immediate -Early Region, *Antimicrobial Agents and Chematherapy* , 37:1945-1954, (1993).

Azuma, Biochemical and Immunological Studies on Cellular Components of Tubercle Bacilli. *Kekkaku*, 69, 9:45-55, (1992).

Ballas ZK et al., Induction of NK act ivity in murine and human cells by CpG motifs in oligodeoxynucleotides and bacterial DNA. J . *Immunol*. 157, 5:1840-1845, (1996).

Bauer M et al., Bacterial CpG -DNA triggers activation and maturation of human CD11c-, CD123+ dendritic cells. *J*. *Immunol*. 166:5000-5007 (2001).

Bauer S et al., H. Bacterial CpG -DNA licenses TLR9. *Curr. Top. Microbiol. Immunol.* 270:145-154 (2002).

Bauer S et al., Human TLR9 confers responsiveness to bacterial DNA via species - specific CpG motifrccognition. *Proc. Natl. Acad. Sci. USA ,* 98: 9237-9242, (2001).

Beignon AS et al., Immunization onto bare skin with synthetic peptides: immunomodulation with a CpG-containing oligodeoxynucleotide and effective priming of influenza virus-specific CD4+ T cells. *Immunology,* 105:204-2012 (2002).

Boggs RT et al., Characterization and modulation of immune stimulation by modified oligonucleotides. *Antisense Nucleic Acid Drug Dev.* 7, 5:461-471, (1997).

Bohle B. CpG motifs as possible adjuvants for the treatment of allergic diseases. *Int. Arch. Allergy Immunol.* 129:198-203, (2002).

Bozza S et al., Vaccination of mice against invasive aspergillosis with recombinant

Aspergillus proteins and CpG oligodeoxynucleotides as adjuvants. *Microbes Infect*. 4:1281-1290, (2002).

Branda RF et al., Amplification of antibody production by phosphorothioat oligodeoxynucleotides. *J. Lab. Clin. Med*. 128, 3:329-338, (1996).

Carlson GA. Postexposure prophylaxis against transmissible spongiform encephalopathies: CpG oligodeoxynucleotides in mice. *Lancet*, 360:184, (2002).

Carpentier et al., Oligodeoxynucleotides containing CpG motifs can induce rejection of a neuroblastoma in mice. *Cancer Res.* 59, 5429-5432, (1999).

Chace JH et al., Bacterial DNA -induced NK cell IFNγ production is dependent on macrophage secretion of IL -12. Clin. *Immunol. Immunopathol.* 84, 185-193, (1997).

Chen W et al., Enhancement of antigen -presenting ability of B lymphoma cells by immunostimulatory CpG-oligonucleotides and anti-CD40 antibody. *Immunol. Lett.* 77:17-23 (2001).

Choudhury BK et al., In vivo role of p38 mitogen-activated protein kinase in mediating the anti-inflammatory effects of CpG oligodcoxynucleotide in murine asthma. *J. Immunol*.169:5955-5961, (2002).

Chu RS et al., CpG oligodeoxynucleotides act as adjuvants that switch on T helper 1 (Th1) immunity, *J. Exp. Med*.186, 1623-1631, (1997).

Chuang TH et al., Toll -like receptor 9 mediates CpG -DNA signaling. *J. Leukoc. Biol.* 71:538-544, (2002).

Cowdery JS et al., Bacterial DNA induces NK cells to produce IFN -gamma in vivo and increases the toxicity of lipopolysaccharides, *J. Immunol*. 156, 12:4570-4575, (1996).

Dalpke AH et al., Phosphodiester CpG oligonucleotides as adjuvants: polyguanosine runs enhance cellular uptake and improve immunostimulative activity of phosphodiester CpG oligonucleotides in vitro and in vivo. *Immunology,* 106:102-1012, (2002).

Davis HL et al., CpG DNA is a potent enhancer of specific immunity in mice immunized with recombinant hepatitis B surface antigen. *J. Immunol*.160, 870-876, (1998).

Decker T et al., Effect of immunostimulato ry CpG-oligonuelcotides in chronic lymphocytic leukemia B cells. Leuk. Lymphoma. 42:301 -307 (2001).

Decker T et al., Immunostimulatory CpG -oligonucleotides cause proliferation, cytokine production, and an immunogenic phenotype in chronic lymphocytic leukemia B cells. *Blood,* 95:999-1006 (2000).

Decker T et al., Peschel C. Immunostimulatory CpG -oligonucleotides induce functional high affinity IL -2 receptors on B -CLL cells: costimulation with IL -2 results in a highly immunogenic phenotype. *Exp. Hematol.* 28:558-68 (2000).

Decker T et al., Sensitization of B -cell chronic lymphocytic leukemia cells to recombinant immunotoxin by immunostimulatory phosphorothioate oligodeoxynucleotides. *Blood,* 99:1320-1326, (2002).

Diwan M et al., Enhancement of immune responses by co -delivery of a CpG oligodeoxynucleotide and tetanus toxoid in biodegradable nanospheres. J. Control

Release. 85:247-62, (2002).

Dumais N et al., Mucosal immunization with inactivated human immunodeficiency virus plus CpG oligodeoxynucleotides induces genital immune responses and protection against intravaginal challenge. *J. Infect. Dis.* 186:1098-1105 (2002).

Elias F et al. Strong Cytosine -Guanosine-Independent Immunostimulation in Humans and Other Primates by Synthetic Oligodeoxynucleotides with PyNTTTTG T Motifs. *J. immunol*, 171: 3697-3704 (2003).

Erb KJ et al. Infection of mice with Mycobacterium bovis -Bacillus Calmette-Guerin (BCG) suppresses allergen-induced airway eosinophilia. *J*. *Exp. Med.* 187, 4:561-569, (1998).

Fattorini L et al., Recombinant GroES in combination with CpG oligodeoxynucleotides protects mice against Mycobacterium avium infection. *J. Med. Microbiol.* 51:1071-1079, (2002).

Gicrynska M et al., Induction of CD8 T -cell-specific systemic and mucosal immunity against herpes simplex virus with CpG-peptide complexes. *J*. *Virol*. 76:6568-6576 (2002).

Gill RF et al., Enhancement of rat tear IgA antibody responses following intranasal immunization with antigen and CpG ODN. *Curr. Eye Res*. 24:228-233, (2002).

Gursel M et al., CpG oligodeoxynucleotides induce human monocytes to mature into functional dendritic cells. *Eur. J. Immunol*. 32:2617-2622 (2002).

Gursel M et al., Differential and competitive activation of human immune cells by distinct classes of CpG oligodeoxynucleotide. *J. Leukoc. Biol.* 71:813-820, (2002).

Hadden J et al., Immunopharmacology, JAMA, 268, 20:2964 -2969, (1992).

Hadden J et al., Immunostimulants. *TIPS*, 141:169-174, (1993).

Halpern MD et al., Bacterial DNA induces murine interferon -gamma production by stimulation of interleukin-12 and tumor necrosis factor-alpha. *Cell Immunol*. 167, 1:72-78, (1996).

Harandi AM et al., A protective role of locally administered immunostimulatory CpG oligodeoxynucleotide in a mouse model of genital herpes infection. *J. Virol*. 77:953-962 (2003).

Hartmann et al., Mechanism and function of a newly identified CpG DNA motif in human primary B cells. *J. Immunol*. 164, 944-952, (2000).

Hartmann G et al., Delineation of a CpG phosphorothioate oligodeoxynucleotide for activating primate immune responses in vitro and in vivo. *J. Immunol.*164*,* 1617-1624, (2000).

Heckelsmiller K et al., Combined dendritic cell - and CpG oligonucleotide based immune therapy cures large murine tumors that resist chemotherapy. Eur. J. Immunol. 32:3235-45, (2002).

Heckelsmiller K et al., Peritumoral CpG DNA elicits a coordinated response of CD8 T cells and innate effectors to cure established tumors in a murine colon carcinoma model. J. Immunol. 169:3892 -3899, (2002).

Horner AA et al., Immunostimulatory sequence oligodeoxynucleotide -based vaccination and immunomodulation: two unique but complementary strategies for the treatment of allergic diseases. *J. Allergy Clin. Immunol*. 110:706-712 (2002).

Horner AA et al., Optimized conjugation ratios lead to allergen immunostimulatory oligodeoxynucleotide conjugates with retained immunogenicity and minimal anaphylactogenicity. *J. Allergy Clin. Immunol.* 110:413-420, (2002).

Homung V et al., Quantitative expression of toll -like receptor 1-10 mRNA in cellular subsets of human peripheral blood mononuclear cells and sensitivity to CpG oligodeoxynucleotides. *J. Immunol.* 168:4531-4537, (2002).

Hussain I et al., Modulation of murine allergic rhinosinusitis by CpG oligodeoxynucleotides. *Laryngoscope*, 112:1819-1826, (2002).

Ioannou XP et al., CpG -containing oligodeoxynucleotides, in combination with conventional adjuvants, enhance the magnitude and change the bias of the immune responses to a herpesvirus glycoprotein. *Vaccine* 21:127-37, (2002).

Ioannou XP et al., The immunogenicity and protective efficacy of bovine herpesvirus 1 glycoprotein D plus Emulsigen are increased by formulation with CpG oligodeoxynucleotides. *J. Virol*. 76:9002-9010, (2002).

Ishii KJ, et al., Potential role of phosphatidylinositol 3 kinase, rather than DNA - dependent protein kinase, in Cp G DNA-induced immune activation. *J. Exp. Med.* 196:269-274 (2002).

Jahrsdorfer B et al., CpG DNA increases primary malignant B cell expression of costimulatory molecules and target antigens. *J. Leukoc. Biol*. 69:81-88 (2001).

Jahrsdorfer B et al., Modulation of malignant B cell activation and apoptosis by bc1-2 antisense ODN and immunostimulatory CpG ODN. *J. Leukoc. Biol*. 72:83-92, (2002).

Jain VV et al., CpG-oligodeoxynucleotides inhibit airway remodeling in a murine model of chronic asthma. *J. Allergy Clin. Immunol*. 110:867-872, (2002).

Jiang W et al., Mucosal immunization with helicobacter, CpG DNA, and cholera toxin is protective. *Infect. Immun.* 71:40-46, (2003).

Joseph A et al., Liposomal immunostimulatory DNA sequence (ISS -ODN): an efficient parenteral and mucosal adjuvant for influenza and hepatitis B vaccines. *Vaccine.* 20:3342-3354 (2002).

Juffermans NP et al., CpG oligodeoxynucleotides enhance host defense during murine tuberculosis. *Infect. Immun.* 70:147-152 (2002).

Jung J et al., Distinct response of human B cell subpopulations in recognition of an innate immune signal, CpG DNA. *J. Immunol.* 169:2368-2373, (2002).

Kadowaki N et al., Distinct CpG DNA and polyinosinic -polycytidylic acid doublestranded RNA, respectively, stimulate CD11c⁻ type 2 dendritic cell precursors and

CD11c⁺ dendritic cells to produce type I IFN. *J. Immunol*., 166,2291- 2295, (2001).

Kadowaki N et al., Subsets of human dendritic cell precursors express different toll - like receptors and respond to different microbial antigens. *J. Exp. Med.* 17;194:863-869 (2001).

Kataoka T et al., Antitumor Activity of Synthetic Oligonucleotides with Sequences from cDNA Encoding Proteins of Mycobacterium bovis BCG. Jpn. J. *Cancer Res*., 83:244-247, (1992).

Kimura T et al., Binding of Oligoguanylate to Scavenger Receptors Is Requiered for Oligonucleotides to Augment NK Cell Activity and Induce IFN. *J. Biochem.* 116, 5:991-994, (1994).

Kitagaki K et al., Immunomodulatory effects of CpG oligodeoxynucleotides on established th2 responses. *Clin. Diagn. Lab. Immunol*. 9:1260-1269, (2002).

Kline JN et al., CpG oligonucleotides can reverse as well as prevent TH2 -mediated inflammation in a murine model of asthma. *J. Invest. Med*., 45, 7:298A, (1997).

Kline JN et al., Immune redirection by CpG oligonucleotides. Conversion of a Th2 response to a Th1 response in a murine model of asthma. *J. Invest. Med*., 45, 3:282A, (1997).

Kline JN et al., Modulation of airway inflammation by CpG oligodeoxynucleotides in a murine model of asthma. *J. immunol.* 15, 2555 -2559, (1998).

Kline JN et al., Treatment of established asthma in a murine model using CpG oligodeoxynucleotides. Am. *J. Physiol. Lung. Cell. Mol. Physiol.* 283:170-179, (2002).

Klinman DM et al., CpG motifs present in bacteria DNA rapidly induce lymphocytes to secrete interleukin 6, interleukin 12, and interferon gamma. *Proc. Natl. Acad. Sci. USA*, 93, 7:2879-2883, (1996).

Krieg AM el al., A Role for Endogenous Retroviral Sequences in the Regulation of Lymphocyte Activation. *J. Immunol.,* 143, 2448-2451, (1989).

Krieg AM et al, Phosphorothioate Oligodeoxynucleotides: Antisense or Anti - Protein?, *Antisense Res. Develop.* 5:241, (1995).

Krieg AM et al. Leukocyte Stimulation by Oligodeoxynucleotides. *Applied Antisense Oligonucleotide Technology,* 431-448, (1998).

Krieg AM et al., CpG motifs in bacterial DNA trigger direct B -cell activation. *Nature*, 374:546-549, (1995).

Krieg AM et al., Oligodeoxynucleotide modifications determine the magnitude of B cell stimulation by CpG motifs. *Antisense Nucleic Acid Drug Dev*., 6, 2:133-139, (1996).

Krieg AM et al., The role of CpG dinucleotides in DNA vaccines. *Trends in Microbiology,* 6, 23-37, (1998).

Krieg AM, An innate immune defense mechanism based on the recognition of CpG motifs in microbial DNA. *J. Lab. Clin. Med*., 128,2:128-33, (1996).

Krieg AM. CpG motifs in bacterial DNA and their immune effects. *Annu. Rev. Immunol*. 20:709-760 (2002).

Krieg AM. GpG motifs in bacterial DNA and their immune effects. An *nu. Rev. Immunol.,* 20, 709-760, (2002).

Krug A et al., Identification of CpG oligo nucleotide sequences with high induction of IFN-alpha/beta in plasmacytoid dendritic cells. *Eur. J. Immunol*. 31:2154-2163 (2001).

Krug A et al., Toll -like receptor expression reveals CpG DNA as a unique microbial stimulus for plasmacytoid dendritic cells which synergizes with CD40 ligand to induce high amounts of IL-12. *Eur. J. Immunol.* 31:3026-3037, (2002).

Kuramoto, et al., Oligonucleotide Sequences Required for Natural Killer Cell Activation. *Jpn. J. Cancer Res.* 83:1128-1131, (1992).

Liang H et al., Activation of human B cells by phosphorothioate oligodeoxynucleotides. *J. Clin. Invest*. 98, 1119-1129, (1996).

Lingnau K et al., Poly-L-arginine synergizes with oligodeoxynucleotides containing CpG-motifs (CpG-ODN) for enhanced and prolonged immune responses and prevents the CpG-ODN-induced systemic release of pro-inflammatory cytokines. *Vaccine.* 20:3498-3508, (2002).

Lipford GB et al., CpG-containing synthetic oligonucleotides promote B and cytotoxic T cell responses to protein antigen: a new class of vaccine adjuvants. *Eur. J. Immunol*., 27:2340-2344, (1997).

Lipford GB et al., Immunostimulatory DNA: sequence -dependent production of potentially harmful or useful cytokines. *Eur. J. Immunol*. 27:3420-3426, (1997).

Macfarlane DE and Manzel L, Antagonism of immun ostimulatory CpG-oligodeoxynucleotides by quinacrine, chloroquine, and structurally related compounds. *J. Immunol*., 160, 3:1122-1131, (1998).

Magone MT et al., Systemic or mucosal administration of immunostimulatory DNA inhibits early and late phases of murine allergic conjunctivitis. *Eur. J. Immunol*. 30, 1841-1850, (2000).

Mariotti S et al., Immunogenicity of anti -Haemophilus influenzae type b CRM197 conjugate following mucosal vaccination with oligodeoxynucleotide containing immunostimulatory sequences as adjuvant. *Vaccine.* 20:2229-2239, (2002).

Matson S et al., Nonspecific suppression of [3H]thymidine incorporation by "control" oligonucleotides. *Antisense Res. Dev* ., 2, 4:325 -330, (1992).

Maurer T et al., CpG-DNA aided cross-presentation of soluble antigens by dendritic cells. *Eur. J. Immunol.* 32:2356-2364.

McCluskie MJ et al., Parenteral and mucosal prime -boost immunization strategies in mice with hepatitis B surface antigen and CpG DNA. *FEMS Immunol. Med. Microbiol.* 32:179-185, (2002).

Messina et al., Stimulation of in vitro Murine Lymphocyte Proliferation by Bacterial DNA. *J. Immunol*., 147, 6:1759-1764, (1991).

Messina, et al., The Influence of DNA Structure on the in vitro Stimulation of Murine Lymphocytes by Natural and Synthetic Polynucleotide Antigens. *Cell. Immunol*., 147:148-157, (1993).

Miconnct I et al., CpG are efficient adjuvants for specific CTL induction against tumor antigen-derived peptide. *J. Immunol*. 168:1212-1218(2002).

Mojcik C et al., Administration of a Phosphorothioate Oligonucleotide Antisense Murine Endogenous Retroviral MCF env Causes Immune Effect in vivo in a Sequence-Specific Manner. *Clin. Immunol. Immunopathology*. 67, 2:130-136, (1993).

Moldovenu GJ et al., CpG DNA, a novel immune enhancer for systemic and mucosal immunization with influenza virus. *Vaccine*.16, 1216-1224, (1998).

O'Hagan DT et al. Synergistic adjuvant activity of immunostimulatory DNA and oil/water emulsions for immunization with HIV p55 gag antigen. *Vaccine*. 20:3389-98, (2002).

Olbrich AR et al., Effective postexposure treatment of retrovirus -induced disease with immunostimulatory DNA containing CpG motifs. *J. Virol*. 76:11397-404, (2002).

Pal S et al., Immunization with the Chlamydia trachomatis mouse pneumonitis major outer membrane protein by use of CpG oligodeoxynucleotides as an adjuvant induces a protective immune response against an intranasal chlamydial challenge. *Infect. Immun.* 70:4812-4817, (2002).

Park Y et al., Cutting Edge: CpG DNA inhibits dendritic cell apoptosis by up - regulating cellular inhibitor of apoptosis proteins through the phosphatidylinositide - 3'-OH kinase pathway. *J. Immunol*. 168:5-8 (2002).

Pisetsky et al., Stimulation of Murine Lymphocyte Proliferation by a Phosphorothioate Oligonucleotide with Antisense Activity for Herpes Simplex Virus. *Life Science,* 54, 101-107 (1994).

Pisetsky, Immunological Consequences of Nucleic Acid Therapy. *Antisense Res. Development*, 5:219-225 (1995).

Pisetsky, The Immunological Properties of DNA. *J. Immunol*., 421-423 (1996).

Rachmilewitz D et al., Immunost imulatory DNA ameliorates experimental and spontaneous murine colitis. *Gastroenterology.* 122:1428-1441, (2002).

Rankin R et al., CpG motif identification for veterinary and laboratory species demonstrates that sequence recognition is highly concerved. *Antisence Nucleic Acid Drug Dev*., 11:333 -340 (2001).

Rankin R et al., CpG-containing oligodeoxynucleotides augment and switch the immune responses of cattle to bovine herpesvirus -1 glycoprotein D. *Vaccine*, 20:3014-3022, (2002).

Rhcc EG et al., Vaccination wit h heat-killed leishmania antigen or recombinant leishmanial protein and CpG oligodeoxynucleotides induces long -term memory CD4+ and CD8+ T cell responses and protection against leishmania major infection. *J. Exp. Med*. 195:1565-1573 (2002).

Rothenfusser S et al., Plasmacytoid dendritic cells: the key to CpG. *Human Immunol.* 63:1111-1119, (2002).

Santeliz JV et al., Amb a 1-linked CpG oligodeoxynucleotides reverse established airway hyperresponsiveness in a murine model of asthma. *J. Allergy Clin. Immunol*. 109:455-462, (2002).

Sato et al., Immunostimulatory DNA Sequences Necessary for Effective Intradermal Gene Immunization. *Science,* 273:352-354, (1996).

Schultz KR et al., Chloroquine prevention of murine MHC -disparate acute graft-versus-host disease correlates with inhibition of splenic response to CpG oligodeoxynucleotides and alterations in T -cell cytokinc production. *Biol. Blood Marrow Transplant,* 8:648-655 (2002).

Schwartz DA et al., CpG motifs in bacterial DNA cause inflammation in the lower respiratory tract. *J. Clin. Invest*., 100, 1:68-73, (1997).

Senuma A et al., Therapeutic effect of CpG motifs on the development of chronic graft-versus-host disease in mice. Cytokine. 20:23 -29, (2002).

Sethi S et al., Postexposure prophylaxis against prion disease with a stimulator of innate immunity. *Lancet,* 360:229-230, (2002).

Sfondrini L et al., Absence of the CD1 molecule up -regulates antitumor activity induced by CpG oligodeoxynucleotides in mice. *J. Immunol*. 169:151-158, (2002).

Shirota H et al., B cells capturing antigen conjugated with CpG oligodeoxynucleotides induce Th1 cells by elaborating IL -12. *J. Immunol*. 169:787-94, (2002).

Sparwasser T et al., Bacterial DNA and immunostimulatory CpG oligodeoxynucleotides trigger maturation and activation of murine dendritic cells. *Eur. J. Immunol*. 28,2045-2054,1998.

Stern BV et al., Vaccination with tumor peptide in CpG adjuvant protects via IFN - gamma-dependent CD4 cell immunity. *J. Immunol*. 168:6099-6105, (2002).

Sweet MJ et al., Colony-stimulating factor-1 suppresses responses to CpG DNA and expression of toll-like receptor 9 but enhances responses to lipopolysaccharide in murine macrophages. *J. Imm*unol. 168:392-399 (2002).

Takauji R et al., CpG-DNA-induced IFN-alpha production involves p38 MAPK - dependent STAT1 phosphorylation in human plasmacytoid dendritic cell precursors. *J. Leukoc. Biol*. 72:1011-1019 (2002).

Takeshita F et al., Cutting edge: Role of Toll -like receptor 9 in CpG DNA-induced activation of human cells. *J. Immunol*. 67:3555-3558, (2001).

Tokunaga T et al. Synthetic Oligonucleotides with Particular Base Sequences from the cDNA Encoding Proteins of Mycobacterium bovis BCG Induce Interferons and Activate Natural Killer Cells. *Microbiol. Immunol*., 36, 1:55-66, (1992).

Tokunaga T et al., A synthetic single -stranded DNA, poly(dG,dC), induces interferon-alpha/beta and -gamma, augments natural killer activity, and suppresses tumor growth. *Jpn. J. Cancer Res*., 79:682-686, (1988).

Van der Stede Y et al., CpG -oligodinucleotides as an effective adjuvant in pigs for intramuscular immunizations. *Vet. Immunol. Immunopathol*. 86:31-41, (2002).

Verthelyi D et al., CpG oligodeoxynucleotides as vaccine adjuvants in primates. *J*. *Immunol*. 168:1659-1663 (2002).

Verthelyi D et al., Human peripheral blood cells differentially recognize and respond to two distinct CPG motifs. *J. Immunol*. 166: 2372-2377, (2001).

Vicari AP et al., Reversal of tumor-induced dendritic cell paralysis by CpG immunostimulatory oligonucleotide and anti -interleukin 10 receptor antibody. *J. Exp. Med*. 196:541-549, (2002).

Vollmer J et al., Highly immunostimulatory CpG -free oligodcoxynuclcotides for activation of human leukocytes. *Antisense Nucleic Acid Drug Dev* .12:165-175, (2002).

Warren TL and Weiner GJ. Synergism between cytosine -guanine oligodeoxynucleotides and monoclonal antibody in the treatment of lymphoma. Semin. *Oncology,* 29:93-97 (2002).

Weeratna R et al., CpG DNA induces stronger immune responses with less toxicity than other adjuvants. *Vaccine*, 18, 1755-1762, (2000).

Wernette CM et al., CpG oligodeoxynucleotides stimulate canine and feline immune cell proliferation. *Vet. Immunol. Immunopathol*. 84:223-236 (2002).

Wooldridge JE et al., Immunostimulatory oligodeoxynucleotides containing CpG motifs enhance the efficacy of monoclonal antibody therapy of lymphoma. *Blood,* 89, 2994-2998,(1997).

Yamamoto S et al., In vitro augmentation of natural killer cell activity and production of interferon alpha/beta and gamma with deoxyribonucleic acid fraction from Mycobacterium bovis BCG. *Jpn. J. Cancer Res*., 79:866-873, (1988).

Yamamoto S. et al., DNA from Bacteria, but Not from Vertebrates, Induces Interferons, Activates Natural Killer Cells, and Inhibits Tumor Growth. *Microbiol. Immunol*. 36, 9:983-997, (1992).

Yamamoto S. et al., Unique Palindromic Sequences in Synthetic Oligonucleotides are Required to Induce INF and Augment INF -Mediated Natural Killer Activity. *J. Immunol*., 148, 12:4072-4076, (1992).

Yamamoto S., Mode of Action Of Oligonucleotide Fraction Extracted From Mycobacterium bovis BCG. *Kekkaku,* 69, 9:29-32, (1994).

Yamamoto T et al., Ability of Oligonuclcotides with Certain Palindromes to Induce Interferon Produtin and Augment Natural Killer Cell Activity Is Associated with Their Base Length. *Antisense Res. Devel*., 4:119-123, (1994).

Yamamoto T et al., Lipofection of Synthetic Oligodeoxyribonucleotide Having a Palindromic Sequence AACGTT to Murine Splenocytes Enhances Interferon Production and Natural Killer Activity. *Microbiol. Immunol.* 38, 10:831-836, (1994).

Yamamoto T et al., Synthetic Oligonucleo tides with Certain Palindromes Stimulate Interferon Production Of Human Peripheric B Lymphocytes in vitro, *Jpn. J. Cancer Res.,* 85:775-779, (1994).

Yi A et al., IFN-.gamma. Promotes IL-6 and IgM Secretion in Response to CpG Motifs in Bacterial DNA and Oligonucleotides. *J. Immunol*. 558-564 (1996).

Yi AK et al. Role of mitogen-activated protein kinascs in CpG DNA-mediated IL-10 and IL-12 production: central role of extracellular signal -regulated kinase in the negative feedback loop of the CpG DNA-mediated Th1 response. *J. Immunol.* 168:4711-4720, (2002).

Yi Ae-Kyung et al., Rapid Immune Activation by CpG Motifs in Bacterial DNA. *J. Immunol*., 5394-5402 (1996).

Yu D et al., Immunomers novel 3' -3'-linked CpG oligodeoxyribonucleotides as potent immunomodulatory agents. *Nucleic Acids Res*. 30:4460-4469. (2002).

Zelenay, S. Et al., Immunomodulatory effects of plasmid DNA and synthetic oligodeoxynucleotides. J. Eur. J. Immunol. 33: 1382-92 (2003).

## Claims

1. An oligonucleotide having the following sequence: TTTTTTTTTTTTTCATTTTGTTTXPu wherein X is G, A or T and Pu is A or G.

2. The oligonucleotide of claim 1 whereas X is T and Pu is G.

3. The oligonucleotide of claim 1 whereas X is t and Pu is A.

4. The oligonucleotide of claim 1 whereas X is G and Pu is G.

5. The oligonucleotide of claim 1 whereas X is G and Pu is A.

6. The oligonucleotide of claim 1 whereas X is A and Pu is G.

7. The oligonucleotide of claim 1 whereas X is A and Pu is A.

8. The oligonucleotide of claim 1 having any kind of modification of the natural (phosphodiester) phosphate backbone.

9. The oligonucleotide of claim 8 having a phosphate backbone modification on the 5' inter-nucleotide linkages.

10. The oligonucleotide of claim 8 having a phosphate backbone modification on the 3' inter-nucleotide linkages.

11. The oligonucleotide of claim 1 wherein at least one of the internucleotide linkages is a phosphorotioate linkage.

12. The oligonucleotide of claim 1 wherein the oligonucleotide is encapsulated in a slow release delivery vehicle.

13. The oligonucleotide of claim 1 wherein the oligonuelcotide is included in a pharmaceutically acceptable carrier.

14. An oligonucleotide having sequence: SEQ ID NO 8 (IMT 700), SEQ ID NO 12 (IMT 713), SEQ ID NO 14 (IMT 718), SEQ ID NO 15 (IMT 719), SEQ ID NO 16 (IMT 720) or SEQ ID NO 17 (IMT 701).

15. A composition comprising at least an oligonucleotide according to claims 1 to 14 and a pharmaceutically acceptable carrier.

16. A composition according to claim 15 further comprising an antigen.

17. A composition according to claim 16 wherein the antigen is selected from the group consisting of viruses, bacteria, fungi, parasites, tumoral cells, toxins, allergens, proteins, glycolipids and polysaccharides.

18. A composition according to claim 16 wherein say antigen is a viral antigen, a bacterial antigen, a human or animal tumoral cell and/or a fungal antigen.

19. A composition according to claim 16 wherein the oligonucleotide is conjugated with one or more antigens.

20. A composition according to claim 16 wherein the antigen is encoded by a plasmid.

21. A composition according to claims 16 to 20 for vaccination of humans or animals.

22. A composition according claim 15 **characterized in that** the oligonucleotide and the antigen are administered contemporaneously, simultaneously or separately.

23. Use of the oligonucleotide of claims 1 to 14 for the manufacture of a medicament for preventing or treating a tumoral disease.

24. Use according to claim 23 **characterized in that** said tumoral disease is selected from Chronic Myelogenous Leukemia, Melanoma, Kaposi's Sarcoma, Multiple Myeloma, Renal Cell Carcinoma, Bladder Cancer, Lung Cancer, Skin Cancer, Breast Cancer, Colon Cancer and Uterus Cancer.

25. Use of the oligonucleotide of claims 1 to 14 for the manufacture of a medicament for preventing or treating an immunological disorder.

26. Use according to claim 25 **characterized in that** said immunological disorder is selected from Allergy, Severe Combined Immunodeficiency, Chronic Granulomatous disease, Acquired Immunodeficiency Disease and Immunodeficiency caused by trauma or infection.

27. Use of the oligonucleotide of claims 1 to 14 for the manufacture of a medicament for inducing dendritic cell activation.

28. Use according to claims 15 to 27 **characterized in that** said medicament is in liquid or lyophilized form.

29. Use according to claims 15 to 27 **characterized in that** said medicament is administered by intramuscular, oral, intranasal, anal, and vaginal or transdermic route.


SEQUENCE LISTING
(1) GENERAL INFORMATION:
(i) APPLICANTS: IMMUNOTECH S.A.
(ii) TITLE OF INVENTION: Oligonucleotides that induce secretion of
GM-CSF
(iii) NUMBER OF SEQUENCES: 17
(iv) CORRESPONDENCE ADDRESS:
(A) ADDRESSEE: Dragotti & Associati
(B) STREET: Galleria San Babila 4/C
(C) CITY: Milano
(D) STATE:
(E) COUNTRY: Italy
(F) ZIP: 20122
(v) COMPUTER READABLE FORM:
(A) MEDIUM TYPE: Floppy disk
(B) COMPUTER: IBM PC compatible
(vi) CURRENT APPLICATION DATA:
(A) APPLICATION NUMBER:
(B) FILING DATE:
(C) CLASSIFICATION:
(vii) ATTORNEY/AGENT INFORMATION:
(A) NAME: Roberto Pistolesi
(B) REGISTRATION NUMBER: 94880

(C) REFERENCE/DOCKET NUMBER: 04NV31E

(viii) TELECOMMUNICATION INFORMATION:

(A) TELEFONE: 02799340

(B) TELEFAX: 02784427

(2) INFORMATION FOR SEQ ID Nº1 (IMT 022):

(i) SEQUENCE CHARACTERISTICS:

(A) LENGTH: 24 base pairs

(B) TYPE: nucleic acid

(C) STRANDEDNESS: single

(D) TOPOLOGY: linear

(ii) MOLECULE TYPE: DNA

(iii) SEQUENCE DESCRIPTION: SEQ ID Nº 1:

TGCTGCAAAAGAGCAAAAGAGCAA

(3) INFORMATION FOR SEQ ID Nº2 (IMT 504):

(i) SEQUENCE CHARACTERISTICS:

(A) LENGTH: 24 base pairs

(B) TYPE: nucleic acid

(C) STRANDEDNESS: single

(D) TOPOLOGY: linear

(ii) MOLECULE TYPE: DNA

(iii) SEQUENCE DESCRIPTION: SEQ ID Nº 2:

TCATCATTTTGTCATTTTGTCATT

(4) INFORMATION FOR SEQ ID Nº3 (IMT 561):
(A) SEQUENCE CHARACTERISTICS:
(A) LENGTH: 24 base pairs
(B) TYPE: nucleic acid
(C) STRANDEDNESS: single
(D) TOPOLOGY: linear
(B) MOLECULE TYPE: DNA
(C) SEQUENCE DESCRIPTION: SEQ ID Nº 3:
GCATCATTTTGTCATTTTGTCATT

(5) INFORMATION FOR SEQ ID Nº4 (IMT 563):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID Nº 4:
TTATCATTTTGTCATTTTGTCATT

(6) INFORMATION FOR SEQ ID Nº5 (IMT 565):
I. SEQUENCE CHARACTERISTICS:
(A) LENGTH: 24 base pairs

(B) TYPE: nucleic acid
(C) STRANDEDNESS: single
(D) TOPOLOGY: linear
II. MOLECULE TYPE: DNA
III. SEQUENCE DESCRIPTION: SEQ ID N ° 5:
TCCTCATTTTGTCATTTTGTCATT
(7) INFORMATION FOR SEQ ID Nº 6 (IMT 518):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID Nº 6:
TCGTCGAAAAGT CGAAAAGTCGAA
(8) INFORMATION FOR SEQ ID Nº7 ( IMT186)
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID Nº 7:

TTTTTTCTTTTTCTCTTTTTCTCT

(9) INFORMATION FOR SEQ ID N° 8 (IMT 700);

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N°8:

TTTTTTTTTTTTCATTTTGTTTTG

(10) INFORMATION FOR SEQ ID N° 9 ( IMT183)

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 9:

TCATCATTTTGTAAAAAAAAAAAA

(11) INFORMATION FOR SEQ ID N°10 ( IMT184)

I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N°10:
TCATCATTTTGTCAAAAAAAAAAA

(12) INFORMATION FOR SEQ ID N°11 ( IMT185)
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N°11:
AAAAAAAAAAAAAAAAAAAAAAAA

(13) INFORMATION FOR SEQ ID N°12 ( IMT186)
I. SEQUENCE CHARACTERISTICS:
(A) LENGTH: 24 base pairs
(B) TYPE: nucleic acid

(C) STRANDEDNESS: single
(D) TOPOLOGY: linear
II. MOLECULE TYPE: DNA
III. SEQUENCE DESCRIPTION: SEQ ID N°12:
GGGGGGGGGGGGGGGGGGGGGGGG

(13) INFORMATION FOR SEQ ID N °12 (IMT 713)
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N°12:
TTTTTTTTTTTTCATTTTGTTTTA

(14) INFORMATION FOR SEQ ID N°13 (IMT 140)
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N°13:

CCCCCCCCCCCCCCCCCCCCCCCC

(15) INFORMATION FOR SEQ ID N°14 (IMT 718)

I. SEQUENCE CHARACTERISTIC S:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N°14:

TTTTTTTTTTTTCATTTTGTTTAA

(16) INFORMATION FOR SEQ ID N°15 (IMT 719)

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N°15:

TTTTTTTTTTTTCATTTTGTTTGA

(17) INFORMATION FOR SEQ ID N°16 (IMT 720)

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID Nº16:

TTTTTTTTTTTTCATTTTGTTTAG

(18) INFORMATION FOR SEQ ID Nº 17 ( IMT701)

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID Nº 17:

TTTTTTTTTTTTCATTTTGTTTGG

SEQUENCE LISTING

<110>  Immunotech SA

<120>  Oligonucleotides that induce secretion of GM-CSF

<130>  04NV31E

<140>  EP04105792.8

<141>  2004-11-16

<160>  17

<170>  PatentIn version 3.1

<210>  1

<211>  24

<212>  DNA

<213>  Artificial Sequence

<220>

<223>  Oligotide that induce the secretion of GM-CSF

<400>  1
tgctgcaaaa gagcaaaaga gcaa                                          24

<210>  2

<211>  24

<212>  DNA

<213>  Artificial Sequence

<220>

<223>  Oligotide that induce the secretion of GM-CSF

<400>  2
   tcatcatttt gtcattttgt catt                                           24

<210>  3

<211>  24

<212>  DNA

<213>  Artificial Sequence

<220>

<223>  Oligotide that induce the secretion of GM-CSF

<400>  3
gcatcatttt gtcattttgt catt                                              24

<210>  4

<211>  24

<212>  DNA

<213>  Artificial Sequence

<220>

<223>  Oligotide that induce the secretion of GM-CSF

<400>  4
ttatcatttt gtcattttgt catt                                              24

<210>  5

<211>  24

<212>  DNA

<213>  Artificial Sequence

<220>

<223>  Oligotide that induce the secretion of GM-CSF

<400>  5
tcctcatttt gtcattttgt catt                                              24

<210>  6

<211>  24

<212>  DNA

<213>  Artificial Sequence

<220>

<223>  Oligotide that induce the secretion of GM-CSF

<400>  6
tcgtcgaaaa gtcgaaaagt cgaa                                              24

<210> 7

<211> 24

<212> DNA

<213> Artificial Sequence

<220>

<223> Oligotide that induce the secretion of GM-CSF

<400> 7
tttttttttt ttcattttgt tttg 24

<210> 8

<211> 24

<212> DNA

<213> Artificial Sequence

<220>

<223> Oligotide that induce the secretion of GM-CSF

<400> 8
   tcatcatttt gtaaaaaaaa aaaa 24

<210> 9

<211> 24

<212> DNA

<213> Artificial Sequence

<220>

<223> Oligotide that induce the secretion of GM-CSF

<400> 9
   tcatcatttt gtcaaaaaaa aaaa 24

<210> 10

<211> 24

<212> DNA

<213> Artificial Sequence

<220>

<223>  Oligotide that induce the secretion of GM-CSF

<400>  10
aaaaaaaaaa aaaaaaaaaa aaaa                                              24

<210>  11

<211>  24

<212>  DNA

<213>  Artificial Sequence

<220>

<223>  Oligotide that induce the secretion of GM-CSF

<400>  11
gggggggggg gggggggggg gggg                                              24

<210>  12

<211>  24

<212>  DNA

<213>  Artificial Sequence

<220>

<223>  Oligotide that induce the secretion of GM-CSF

<400>  12
tttttttttt ttcattttgt ttta                                              24

<210>  13

<211>  24

<212>  DNA

<213>  Artificial Sequence

<220>

<223>  Oligotide that induce the secretion of GM-CSF

<400>  13
cccccccccc cccccccccc cccc                                              24

<210>  14

<211>  24

<212>  DNA

<213>  Artificial Sequence

<220>

<223>  Oligotide that induce the secretion of GM-CSF

<400>  14
tttttttttt ttcattttgt ttaa                                              24

<210>  15

<211>  24

<212>  DNA

<213>  Artificial Sequence

<220>

<223>  Oligotide that induce the secretion of GM-CSF

<400>  15
tttttttttt ttcattttgt ttga                                              24

<210>  16

<211>  24

<212>  DNA

<213>  Artificial Sequence

<220>

<223>  Oligotide that induce the secretion of GM-CSF

<400>  16
tttttttttt ttcattttgt ttag                                              24

<210>  17

<211>  24

<212>  DNA

<213>  Artificial Sequence

<220>

<223>  Oligotide that induce the secretion of GM-CSF

<400>  17
tttttttttt ttcattttgt ttgg                                              24